# EUROPÄISCHE PATENTSCHRIFT

(11) **EP 2 252 351 B1**
(45) Veröffentlichungstag und Bekanntmachung des Hinweises auf die Patenterteilung: **03.12.2014**
(21) Anmeldenummer: 09711170.2
(22) Anmeldetag: 04.02.2009
(51) Int. Cl.: A61M 5/315, A61M 5/20, A61M 5/24

(54) **VERABREICHUNGSVORRICHTUNG MIT HALTEEINRICHTUNG**
ADMINISTERING APPARATUS COMPRISING A HOLDING DEVICE
SYSTÈME D'ADMINISTRATION DE PRODUIT À DISPOSITIF DE RETENUE

(30) Priorität: 11.02.2008 CH 188082008
(43) Veröffentlichungstag der Anmeldung: 24.11.2010
(73) Patentinhaber: Tecpharma Licensing AG, 3400 Burgdorf (CH)
(72) Erfinder: HIRSCHEL, Jürg, CH-5000 Aarau (CH); KÄNEL-JOST, Céline, CH-3414 Oberburg (CH); MOSER, Ulrich, CH-3412 Heimiswil (CH); TSCHIRREN, Markus, CH-3422 Kirchberg (CH)
(86) Internationale Anmeldenummer: PCT/CH2009/000041
(87) Internationale Veröffentlichungsnummer: WO 2009/100549

(56) Entgegenhaltungen:
- WO-A-2004/054644
- WO-A-2008/005315
- DE-A1-102005 038 933
- US-A- 2 752 918
- US-A- 2 960 087

## Beschreibung

### Technisches Gebiet

Die vorliegende Erfindung betrifft eine Vorrichtung zur Verabreichung eines fluiden Produkts, wie etwa medizinische, pharmazeutische oder kosmetische Wirkstoffe. Insbesondere betrifft die Erfindung ein Injektionsgerät zur Verabreichung eines fluiden Produkts in ein Körpergewebe.

### Stand der Technik

Verabreichungsgeräte wie sie die Erfindung betrifft umfassen im allgemeinen mehrere Bauteile, die zum einen eine Verabreichungsmechanik bilden und zum anderen eine Aufnahme für das fluide Produkt vorsehen. Zur Verabreichung des fluiden Produkts wird ein Antriebsglied innerhalb der Verabreichungsvorrichtung bewegt, bzw. verschoben, um das fluide Produkt aus der Verabreichungsvorrichtung auszuschütten. Dabei kann die Verabreichungsmechanik aus einer Vielzahl von einzelnen zu einander beweglichen Bauteilen bestehen. Weiter kann die Aufnahmevorrichtung für das fluide Produkte mehrere Komponenten umfassen, wie etwa eine GlasKarpule in der ein Wirkstoff aufgenommen ist und einen Karpulenhalter in dem die Karpule untergebracht ist. Zum einen existieren Verabreichungsvorrichtungen, bei welchen die Aufnahmevorrichtung von der Verabreichungsmechanik abgekoppelt werden kann, um z.B. eine neue Karpule einzusetzen, nachdem eine alte entleerte Karpule entsorgt wurde. Die Aufnahmevorrichtung kann mit der neuen Karpule wieder an die Verabreichungsmechanik angekoppelt werden. Zum anderen existieren Einweg-Verabreichungsvorrichtungen, bei welchen nach dem Entleeren der Aufnahmevorrichtung die gesamte Verabreichungsvorrichtung mitsamt der Verabreichungsmechanik und der Aufnahmevorrichtung entsorgt werden.

Für beide Arten von Verabreichungsvorrichtungen ist es für eine exakte Dosierung bei der Ausschüttung des fluiden Produkts erforderlich, dass die einzelnen Bauteile der Verabreichungsvorrichtung in einer definierten Position zueinander angeordnet sind. Besteht ein zu grosses Spiel zwischen den einzelnen Bauteilen, ist es möglich, dass beim Vorschub des Antriebsglieds nicht eine definierte, der gewünschten Dosis entsprechende Strecke zur Ausschüttung des fluiden Produkts zurückgelegt wird. Die Dosierstrecke ist in diesem Fall nicht präzise und es kann zu Über- oder Unterdosierungen kommen. Bei Verabreichungsvorrichtungen der beschriebenen Art ist es daher wesentlich, die einzelnen Bauteile in einer definierten Position zu einander zu halten.

Aus der DE 10 2005 032 705 A1 ist ein Injektionsgerät bekannt, dass ein Gehäuse zur Aufnahme einer Verabreichungsmechanik und einen Karpulenhalter in dem eine Karpule einsetzbar ist, aufweist. Der Karpulenhalter ist an das Gehäuse angeschlossen. Ein Antriebsglied der Verabreichungsmechanik greift an einem Stopfen in der Karpule an, um bei einem Vorschub des Antriebsglieds den Stopfen in der Karpule vorzuschieben und den fluiden Wirkstoff mittels einer aufgesetzten Injektionsnadel aus der Karpule auszuschütten. Das Gehäuse des Injektionsgeräts, bzw. ein gehäusefestes Teil des Injektionsgeräts weist eine Kunststofffeder auf, die relativ zum Gehäuse feststeht. Wird der Karpulenhalter in das Gehäuse eingesetzt, greift die Kunststofffeder an das proximale Ende der Karpule an und drückt durch ihre Vorspannung die Karpule gegen eine distal am Karpulenhalter angeordnete Schulter. Mittels der gehäusefesten Kunststofffeder wird die Karpule in dem Karpulenhalter gehalten und bewegt sich nicht ungewollt relativ zum Karpulenhalter. Aufgrund der Kunststofffeder vergrössert sich die gesamte Baulänge des Injektionsgeräts und bei der Herstellung des Injektionsgeräts ist ein zusätzlicher Montageschritt zum Einbringen der Kunststofffeder notwendig.

Eine weitere aus dem Stand der Technik bekannte Vorrichtung zur Verabreichung eines fluiden Produkts ist im Dokument WO 2008/005315 A offenbart. Der Oberbegriff des Anspruchs 1 is auf diesem Dokument basiert.

Es ist eine Aufgabe der vorliegenden Erfindung, eine Vorrichtung zur Verabreichung eines fluiden Produkts zu schaffen, bei welcher Bauteile der Vorrichtung eine definierte Position zu zueinander einnehmen, wobei die Vorrichtung mit wenigen Bauteilen herstellbar und einfach in der Montage ausgestaltet ist.

Die Aufgabe der Erfindung wird durch eine Vorrichtung zur Verabreichung eines fluiden Produkts gemäss Patentanspruch 1 erfüllt. Vorteilhafte Ausgestaltungen der erfindungsgemässen Verabreichungsvorrichtungen gehen aus den Unteransprüchen hervor.

### Darstellung der Erfindung

Gemäss der vorliegenden Erfindung wird eine Vorrichtung zur Verabreichung eines fluiden Produkts vorgesehen, die ein Antriebsglied umfasst, das zur Verabreichung des fluiden Produkts in der Verabreichungsvorrichtung beweglich ist, wobei das Antriebsglied eine Halteeinrichtung oder Teile einer Halteeinrichtung aufweist, die wenigstens ein weiteres Element der Verabreichungsvorrichtung in einer definierten Position in der Verabreichungsvorrichtung hält.

Bei der Verabreichungsvorrichtung wird das Ende, an dem das fluide Produkt aus der Vorrichtung austritt als distales Ende und das gegenüberliegende Ende als proximales Ende bezeichnet.

Das Antriebsglied kann ein Bestandteil einer Verabreichungsmechanik sein, und z.B. im Wesentlichen die Form einer Kolbenstange aufweisen. Die Kolbenstange kann z.B. als Zahnstange oder auch als Gewindestange ausgebildet sein. Das Antriebsglied kann manuell betätigt werden oder auch von einem Federelement wie etwa einer Spiralfeder oder Schraubenfeder angetrieben werden. Natürlich ist es auch möglich, dass die Verabreichungsmechanik eine Dosiervorrichtung und eine Anzeigevorrichtung aufweist. Das Antriebsglied ist relativ zur Verabreichungsvorrichtung beweglich, insbesondere ist das Antriebsglied entlang einer Längsachse das Antriebsglied relativ zur Verabreichungsvorrichtung verschiebbar. Es ist auch eine kombinierte Dreh- und Vorschubbewegung denkbar. Vorzugsweise weist die Verabreichungsvorrichtung ein Gehäuse auf, in dem das Antriebsglied beweglich gelagert ist.

Erfindungsgemäss weist das Antriebsglied eine Haltevorrichtung auf, die wenigstens ein weiteres Element der Verabreichungsvorrichtung hält. Ein solches weiteres Element kann z.B. eine Aufnahmevorrichtung zur Aufnahme des fluiden Produkts oder zur Aufnahme eines Behälters für das fluide Produkt sein. Ferner können verschiedene Elemente der Verabreichungsmechanik, einer Dosiermechanik oder einer Anzeigeneinrichtung ein solches weiteres Element darstellen oder beinhalten.

Ferner kann erfindungsgemäss das Antriebsglied nur Teile einer Halteeinrichtung aufweisen. In diesem Fall ist neben dem Haltemittel, welches das Antriebsglied aufweist, wenigstens ein weiteres

Halteelemente vorgesehen. So kann z.B. das Haltemittel des Antriebsglieds ein Element in einer ersten Richtung halten, das heisst Bewegungen des Elements relativ zu anderen Komponenten der Verabreichungsvorrichtung in einer ersten Richtung blockieren. Ein weiteres Halteelement an einer anderen Komponente der Verabreichungsvorrichtung kann derart ausgebildet sein, dass das Element in einer zweiten Bewegungsrichtung blockiert wird. Mit dem Begriff Halten ist im Allegemeinen ein Sichern oder Blockieren einer Bewegung des zu haltenden Elements relativ zu anderen Komponenten der Verabreichungsvorrichtung zu verstehen. Beispielsweise kann eine - erfindungsgemässe Halteeinrichtung eine Aufnahmevorrichtung für das fluide Produkt relativ zu einem Gehäuse der Verabreichungsvorrichtung halten. Oder es wird eine Karpule mit dem fluiden Produkt innerhalb einer Aufnahmevorrichtung gehalten.

Das Antriebsglied und die Halteeinrichtung sind fest miteinander verbunden. Vorzugsweise sind Antriebsglied und Halteeinrichtung einstückig ausgebildet.

Die erfindungsgemässe Halteeinrichtung des Antriebsglieds hält das wenigstens eine weitere Element der Verabreichungsvorrichtung in einer definierten Position in der Verabreichungsvorrichtung. Die definierte Position wird durch die örtliche Anordnung des zu haltenden Elements relativ zu den anderen Komponenten der Verabreichungsvorrichtung bestimmt. Es ist daher möglich, dass das zu haltende Element gegenüber einer ersten Komponente eine definierte, gesicherte Position einnimmt, während das zu haltende Element zu einer zweiten Komponente unterschiedliche Positionen einnehmen kann. Beispielsweise kann eine Karpule in einem Karpulenhalter der Verabreichungsvorrichtung in einer definierten Position gehalten werden, während sich der Karpulenhalter relativ zum Gehäuse der Verabreichungsvorrichtung bewegt.

Vorzugsweise hält die Halteeinrichtung in einer Ausgangsposition des Antriebsglieds und in einer Vorschubposition des Antriebsglieds, in der das Antriebsglied relativ zu dem wenigstens einen weiteren Element gegenüber der Ausgangsposition verschoben ist, das Element in der definierten Position. Beispielsweise wird eine Karpule in einem Karpulenhalter von dem Antriebsglied gehalten, sowohl in einer Ausgangsposition des Antriebsglieds als auch in einer Vorschubposition, in der das Antriebsglied relativ zur Karpule und zum Karpulenhalter verschoben ist.

In einer bevorzugten Ausführungsform weist die Halteeinrichtung von dem Antriebsglied abragende Haltearme auf. Die Haltearme können in der Ausgangsposition des Antriebsglieds von einer Längsachse des Antriebsglieds seitlich abragen und sie können flexibel beweglich sein. Wird das Antriebsglied in der Verabreichungsvorrichtung in die Vorschubposition bewegt, können die Haltearme vorteilhafterweise flexibel bewegt werden um ihre Haltefunktion weiterhin wahrnehmen zu können. Beispielsweise können sie flexibel zur Längsachse hin oder auch von ihr weg bewegt werden.

Das Halten der Halteeinrichtung kann zum einen durch wenigstens einen axialen Anschlag gegeben sein, an dem das wenigstens eine weitere Element der Verabreichungsvorrichtung anschlägt. Ferner kann das Halten auch durch eine Klemmpassung, bzw. einen Klemmsitz, mit dem wenigstens einen weiteren Element erfolgen. Vorzugsweise hält die Halteeinrichtung in der Ausgangsposition des Antriebsglieds das wenigstens eine weitere Element durch wenigstens einen axialen Anschlag des Elements an der Halteeinrichtung, bzw. den Haltearmen, und in einer Vorschubposition des Antriebsglieds erfolgt das Halten der Halteeinrichtung, bzw. der Haltearme, durch eine Klemmpassung mit dem weiteren Element der Verabreichungsvorrichtung. Es ist auch möglich, dass zusätzlich zur Klemmpassung ein Halteanschlag wirkt.

Ferner kann das Antriebsglied das wenigstens eine weitere Element in der Ausgangsposition durch wenigstens einen ersten axialen Anschlag und in der Vorschubposition durch zumindest einen zweiten axialen Anschlag halten. Beim Übergang von der Ausgangsposition in die Vorschubposition findet zum Halten des wenigstens einen weiteren Elements daher ein Übergang vom ersten Halteanschlag zum zweiten Halteanschlag statt.

Vorzugsweise Weist der wenigstens eine Anschlag, insbesondere der erste axiale Anschlag, eine schräge Fläche auf, die in distaler Richtung zur Längsachse geneigt ist. Die schräge Fläche ermöglicht es, dass das wenigstens eine Element am Anschlag abgleitet. In dieser Weise kann z.B. der Übergang von dem wenigstens einen axialen Anschlag zum Halten durch eine Klemmpassung oder zu einem zweiten axialen Anschlag erfolgen.

Gemäss einem Ausführungsbeispiel der vorliegenden Erfindung weist die Verabreichungsvorrichtung ein Gehäuse und eine Aufnahmevorrichtung zur Aufnahme eines Behälters für das fluide Produkt auf, wobei die Aufnahmevorrichtung mit dem Gehäuse verbunden oder verbindbar ist und die Halteeinrichtung hält den Behälter in einer definierten Position in der Aufnahmevorrichtung. Vorzugsweise ist das Antriebsglied durch eine Kolbenstange gegeben, welche an ihrem distalen Ende zwei relativ zur Längsachse abgespreizte Haltearme als Halteeinrichtung aufweist. Zur Verabreichung eines fluiden Produkts aus der Verabreichungsvorrichtung wird das Antriebsglied relativ zum Gehäuse und der Aufnahmevorrichtung verschoben.

Vorzugsweise schlagen die Haltearme der Halteeinrichtung in der Ausgangsposition des Antriebsglieds an einem proximalen Ende des Behälters an. Wird der Behälter beispielsweise durch eine hülsenartige GlasKarpule gebildet, können die Haltearme am Rand der Karpule anschlagen. Das distale Ende der Haltearme bildet einen axialen Anschlag für die Karpule. Sie weisen schräge Flächen auf, die in distaler Richtung zur Längsachse hin geneigt sind. Wird nun das Antriebsglied relativ zur Aufnahmevorrichtung vorgeschoben können die Haltearme der Halteeinrichtung am Rand der Karpule abgleiten und an einem Stopfen in dem Behälter anschlagen. Dabei können die abgespreizten Haltearme flexibel in Richtung der Längsachse des Antriebsglieds nach Innen in den Behälter bewegt werden. Gleichzeitig ist es möglich, dass die Haltearme an der inneren Behälterwand anliegen und eine Klemmpassung bilden. Die flexiblen Haltearme der Halteeinrichtung dienen in diesem Fall gleichzeitig der Halterung des Behälters in der Aufnahmevorrichtung und als Vorschubstössel zum Vorschub der Stopfen in dem Behälter für eine Verabreichung des fluiden Produkts. Grundsätzlich ist es jedoch auch möglich, die Haltearme seitlich und gegenüber dem distalen Ende des Antriebsglieds zurückversetzt an dem Antriebsglied anzuordnen, so dass sie in einer Vorschubposition seitlich an dem Antriebsglied anliegen können, wobei der Vorschubstössel von dem vorderen Ende des Antriebsglieds gebildet wird. Die Halteeinrichtung des Antriebsglieds sichert in dieser Ausführungsform den Behälter für das fluide Produkt innerhalb der Aufnahmevorrichtung gegen eine Bewegung in proximaler Richtung aus der Aufnahmevorrichtung heraus.

Es ist auch möglich, die Halteeinrichtung durch weitere Halteelemente zu ergänzen. Beispielsweise kann die Aufnahmevorrichtung einen Gegenanschlag aufweisen, an dem der Behälter in distaler Richtung anschlägt. In diesem Fall ist der Behälter innerhalb der Aufnahmevorrichtung auch gegen eine Bewegung in distaler Richtung gesichert.

Gemäss einer anderen Ausführungsform der vorliegenden Erfindung dient die Halteeinrichtung des Antriebsglieds dem Halten des Behälters, während die Aufnahmevorrichtung relativ zum Antriebsglied und dem Gehäuse bewegt wird, wobei das Antriebsglied relativ zum Gehäuse in Ruhe bleibt. Ein solcher Fall tritt beispielsweise auf bei der Verwendung von ZweikammerKarpulen, bei welchen in einer ersten Kammer ein Lösungsmittel und in einer zweiten Kammer ein trockener, bzw. lyophilisierter Wirkstoff untergebracht sind. Die beiden Kammern sind durch zwei Stopfen und die Karpulenwand begrenzt. Zum Abmischen des Wirkstoffes mit dem Lösungsmittel werden die Stopfen innerhalb der Karpule derart verschoben, dass der die Kammern trennende Stopfen einen Bypass freigibt, so dass das Lösungsmittel aus der einen Kammer in die Wirkstoffkammer fliessen kann. Der Vorschub der Stopfen kann z.B. die Zweikammer-Karpule in der Aufnahmevorrichtung durch Eindrehen oder Vorschieben der Aufnahmevorrichtung in das Gehäuse der Verabreichungsvorrichtung erfolgen. Während dem Eindrehen oder Einschieben der Aufnahmevorrichtung werden die Stopfen durch den Widerstand des Antriebsglieds innerhalb des Behälters verschoben. Das Antriebsglied bleibt dabei relativ zum Gehäuse in Ruhe.

Bei der vorliegenden Erfindung ist kein gesondertes Halteteil, wie z.B. eine Haltefeder zur Fixierung von Komponenten der Verabreichungsvorrichtung notwendig. Das ohnehin für die Verabreichungsmechanik erforderliche Antriebsglied übernimmt bei einer Verabreichungsvorrichtung gemäss der Erfindung die Haltefunktion. Es sind keine zusätzlichen Bauteile erforderlich, welche das Volumen der Verabreichungsvorrichtung erhöhen und die Montage erschweren. Ferner kann die erfindungsgemässe Halteeinrichtung ihre Haltefunktion auch dann erfüllen, wenn das zu haltende Element relativ zur Halteeinrichtung, bzw. zum Antriebsglied bewegt wird.

### Kurze Beschreibung der Zeichnungen

Bevorzugte Ausführungsbeispiele der Erfindung werden nachfolgend Anhand der Figuren erläutert. An den Ausführungsbeispielen offenbar werdende Merkmale bilden je einzeln und in jeder Kombination die Gegenstände der Ansprüche und auch die vorstehend beschriebenen Ausgestaltungen in vorteilhafter Weise weiter. Es zeigen:
- Figur 1: eine Verabreichungsvorrichtung in einem Ausgangszustand,
- Figur 2: die Verabreichungsvorrichtung in einem abgemischten Zustand,
- Figur 3: die Verabreichungsvorrichtung in einem entlüfteten Zustand,
- Figur 4: die Verabreichungsvorrichtung in einem ausgelösten Zustand,
- Figur 5: die Verabreichungsvorrichtung in einem Zustand nach der Ausschüttung eines Produkts,
- Figur 6a: Schnittzeichnung einer weiteren Ausführungsform einer Verabreichungsvorrichtung mit einer Halteeinrichtung in einer Ausgangsposition und
- Figur 6b: Detailansicht der weiteren Ausführungsform der Verabreichungsvorrichtung in einer Vorschubposition.

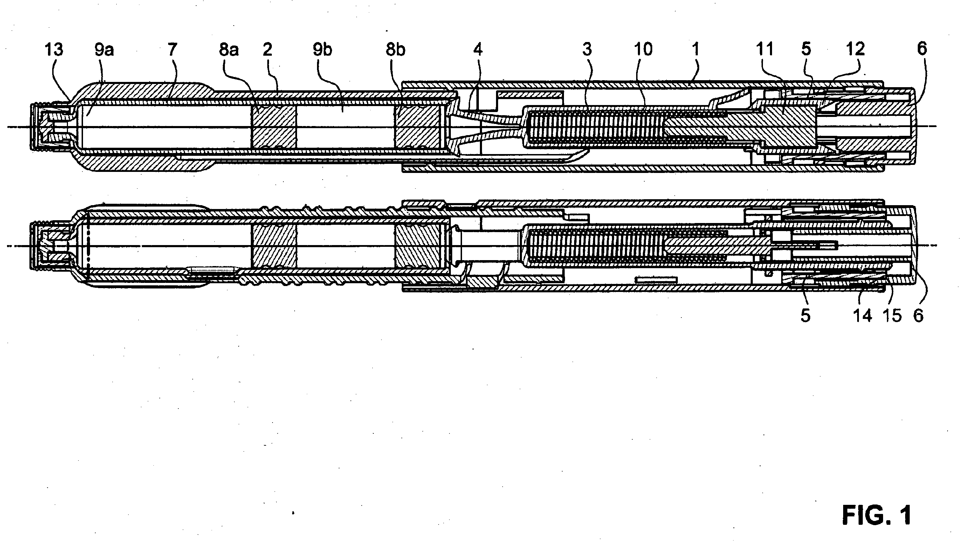

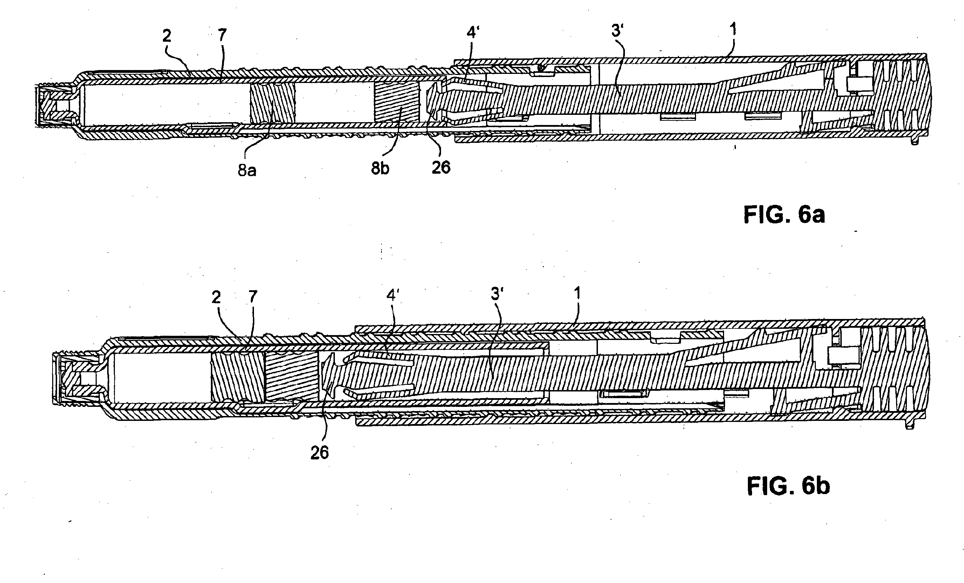

Die Figuren 1 bis 5 zeigen eine erste Ausführungsform einer Verabreichungsvorrichtung gemäss der vorliegenden Erfindung mit einer Halteeinrichtung zum Halten eines Elements der Verabreichungsvorrichtung. In den Figuren 6a und 6b ist eine zweite Ausführungsform einer Halteeinrichtung zum Halten eines Elements der Verabreichungsvorrichtung gezeigt. Die Figuren 1 bis 5 sind jeweils in zwei Ansichten gezeigt, wobei die zweite, untere Ansicht gegenüber der ersten, oberen Ansicht um 90° gedreht ist.

Das Verabreichungsgerät gemäss der ersten Ausführungsform verwendet eine Zweikammer-Karpule als Behälter für ein fluides Produkt. Die Ausschüttung des fluiden Produkts aus der Karpule erfolgt durch den Vorschub eines Antriebsglieds mittels einer Ausschüttfeder. Es erfolgt daher eine automatische Ausschüttung des fluiden Produkts, sobald die Ausschüttfeder aktiviert wird. Die Verabreichungsvorrichtung weist eine fixe Dosierung auf, das heisst, das Ausschüttvolumen ist fest vorgegeben. Der Vorschub des Antriebsglieds ist daher ebenfalls fest vorgegeben und kann nicht individuell eingestellt werden. Das Verabreichungsgerät ist nach einer einzigen Ausschüttung blockiert und wird nach der Ausschüttung entsorgt.

Für einen Fachmann ist es klar, dass eine Halteeinrichtung gemäss der vorliegenden Erfindung gleichwohl bei wieder verwendbaren Verabreichungsgeräten, bei Geräten mit individueller Dosierung oder manueller Ausschüttung und auch bei Geräten mit Einkammer-Karpulen vorteilhaft eingesetzt werden kann.

Das Verabreichungsgerät gemäss einer ersten Ausführungsform der vorliegenden Erfindung weist ein Gehäuse 1, eine Aufnahmevorrichtung zur Aufnahme des fluiden Produkts in Form eines Karpulenhalters 2, ein Antriebsglied 3 mit einer Halteeinrichtung in Form von Haltearmen 4, eine Blockiereinrichtung in Form eines Blockierrings 5 und ein Betätigungselements in Form eines Auslöseknopfes 6 auf.

In dem Karpulenhalter 2 ist eine Zweikammer-Karpule 7 untergebracht. Die Zweikammer-Karpule weist einen ersten Stopfen 8a und einen zweiten Stopfen 8b auf. Der zweite Stopfen 8b schliesst die Zweikammer-Karpule am proximalen Ende ab. Am distalen Ende weist die Zweikammer-Karpule eine Verjüngung auf, deren Öffnung durch eine Membran abgeschlossen ist. Die Membran kann durch eine Kanüle einer Injektionsnadeleinheit durchstochen werden. Die Injektionsnadeleinheit wird in den Figuren nicht dargestellt. Zwischen der Membran und dem ersten Stopfen 8a ist eine erste Kammer 9a ausgebildet, in welcher ein trockener bzw. lyophilisierter Wirkstoff untergebracht wird (nicht dargestellt). Zwischen dem ersten Stopfen 8a und dem zweiten Stopfen 8b wird eine zweite Kammer gebildet, in welcher das Lösungsmittel für den Wirkstoff gelagert wird.

Das Antriebsglied 3 ist hülsenartig ausgebildet. Im inneren des Antriebsglieds 3 ist eine Antriebsfeder 10 angeordnet, die zwischen einem distalen Anschlag am Hülsenboden des Antriebsglieds und einem proximalen Anschlag an einem gehäusefesten Element 11 eingespannt ist. Im Ausgangszustand der Figur 1 wird das Antriebsglied 3 relativ zum Gehäuseelement 11 durch Schnapparme 12 gehalten, welche hinter einem Anschlag des Gehäuseelements 11 lösbar einschnappen. Am distalen Ende des Antriebsglieds 3 sind die Haltearme 4 derart angebracht, dass sie in dieser Ausgangsposition von einer Längsachse des Abtriebsglied seitlich abragen. In der gezeigten Ausführungsform sind zwei Haltearme dargestellt, die zueinander ausgespreizt sind. Es können natürlich auch drei oder mehr derartige Haltearme 4 vorgesehen werden. In dem Ausgangszustand in Figur 1 stossen die Haltearme 4 mit ihrem distalen Ende an eine proximale Kante der Karpule 7. Die Haltearme 4 drücken die Karpule 7 gegen eine Schulter 13 des Karpulenhalters 2. Die Karpule 7 wird daher von der Halteeinrichtung in Form der Haltearme 4 in einer definierten Position relativ zum Karpulenhalter 2 gehalten. Eine hin und her Bewegung in proximaler oder distaler Richtung der Karpule im Halter wird dadurch unterbunden.

Der Blockierring 5 befindet sich im Ausgangszustand der Figur 1 in einer Blockierposition, in der er eine Betätigung des Auslöseknopfes 6 blockiert, d.h. der Auslöseknopf 6 kann nicht in Längsrichtung in das Gehäuse 1 eingedrückt werden. Hierfür weist der Blockierring 5 einen Blockieranschlag 14 auf, der an einem Gegenanschlag 15 am Auslöseknopf 6 ansteht. Aufgrund des Aneinanderstossens des Blockieranschlags 14 des Blockierrings 5 und des Gegenanschlags 15 des Auslöseknopfes 6 kann der Auslöseknopf 6 nicht betätigt werden, das heisst er kann nicht relativ zum Gehäuse entlang der Längsachse in das Gehäuse eingedrückt werden. Hierfür ist der Blockierring 5 relativ zum Gehäuse in Längsrichtung festgelagert, kann jedoch relativ zum Gehäuse verdreht werden. Der Blockieranschlag 14 kann z.B. durch Rippen oder Nocken am Blockierring oder durch die proximale Kante des Blockierrings 5 realisiert werden.

Der Blockierring 15 ist hülsenartig ausgebildet und umgibt die Schnapparme 12 des Antriebsglieds 3. Im Ausgangszustand der Figur 1 liegt die Innenumfangsfläche des Blockierrings 5 derart aussen an den Schnapparmen 12 an, dass diese nicht aus ihrer Verschnappung hinter dem Gehäuseelement 11 freigegeben werden können. Der Blockierring 5 blockiert somit zum einen eine Betätigung des Auslöseknopfes 6 und zum anderen eine Freigabe der Schnapparme 12. Der Ausgangszustand entspricht einem Auslieferungszustand, wie das Verabreichungsgerät an einen Anwender abgegeben wird. Eine Betätigung des Verabreichungsgeräts ist in diesem Zustand nicht möglich.

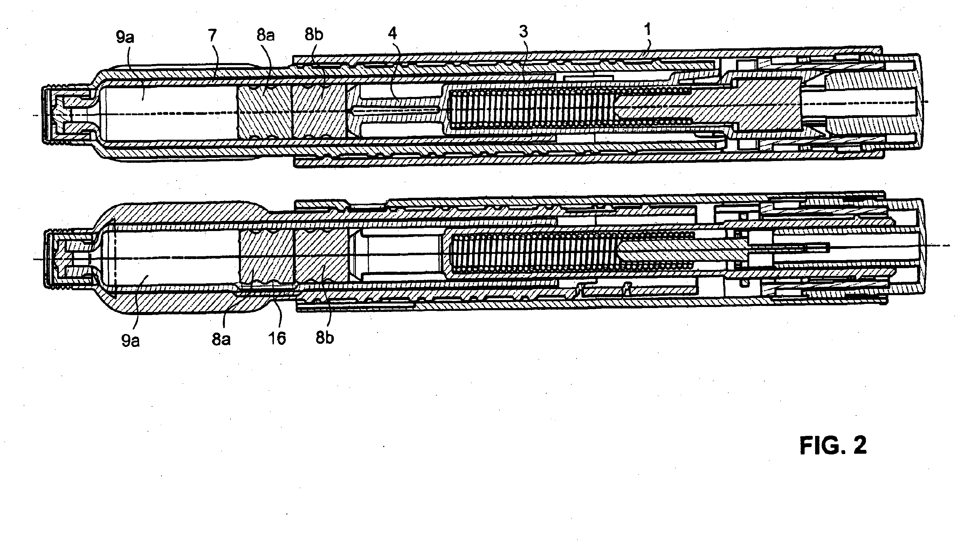

In Figur 2 ist das Verabreichungsgerät in einem abgemischten Zustand gezeigt, in dem der Wirkstoff der Kammer 9a der Zweikammer-Karpule 7 mit dem Lösungsmittel der Kammer 9b abgemischt ist. Die Beendigung des Abmischens kann durch ein taktiles, akustisches oder visuelles Signal angezeigt werden. Wie in Figur 2 gezeigt ist wurden zum Abmischen die Stopfen 8a und 8b innerhalb der Karpule 7 verschoben, bis der Stopfen 8a an einem Bypass 16 zu liegen kommt, durch den das Lösungsmittel in die Kammer 9a fliessen kann und der Stopfen 8b an dem Stopfen 8a zu liegen kommt. Zum Vorschub der Stopfen wird der Karpulenhalter 2 in das Gehäuse 1 eingeschraubt, so dass das Antriebsglied, dass in diesem Zustand relativ zum Gehäuse in Ruhe ist, die Stopfen 8a und 8b relativ zur Karpule 7 verschiebt. Zum Einschrauben des Karpulenhalters ist an der Innenseite des Gehäuses ein Innengewinde und an der Aussenseite des Karpulenhalters ein Aussengewinde vorgesehen.

Wie in Figur 2 zu sehen ist, sind die Haltearme 4 an der proximalen Kante der Karpule 7 abgerutscht und wurden radial nach innen in Richtung der Längsachse des Antriebsglieds bewegt. Hierfür weisen die Enden der Haltearme 4 schräge Flächen auf, entlang welcher die Haltearme 4 nach innen abgelenkt werden, sobald die proximale Kante der Karpule 7 mit ausreichender Kraft gegen die schrägen Flächen gedrückt wird, wie es beim Einschrauben des Karpulenhalters 2 in das Gehäuse 1 der Fall ist. Die Haltearme 4 bewegen sich dabei nach innen aufeinander zu und bilden einen Stössel für den Stopfen 8b der Karpule 7. Durch den Anschlag der Haltearme 4 an dem Stopfen 8b, wird die Karpule 7 weiterhin in ihrer definierten Position in dem Karpulenhalter 2 gehalten, während die Stopfen 8a und 8b innerhalb der Karpule 7 verschoben werden. Unabhängig davon bilden die Haltearme 4 mit der Innenwand der Karpule 7 eine Klemmpassung, bzw. einen Klemmsitz, da sie eine Vorspannung in radialer Richtung nach aussen aufweisen, seitdem sie radial nach innen verbogen wurden. Aus die Klemmpassung dient dem Halten der Karpule in einer definierten Position in dem Karpulenhalter. Die erfindungsgemässe Halteeinrichtung, bzw. der Teil der Halteeinrichtung, des Antriebsglieds kann daher zwei Funktionen übernehmen, erstens das halten der Karpule und zweitens als Vorschubanschlag des Antriebsglieds zur Verabreichung des fluiden Produkts dienen.

Nach dem Abmischen der Zweikammer-Karpule kann es notwendig sein, die Kammer 9a mit dem gelösten Wirkstoff zu entlüften, bevor der Wirkstoff injiziert werden kann. Hierfür wird eine Injektionsnadeleinheit auf den Karpulenhalter 2 am distalen Ende derart aufgebracht, dass eine Kanüle die Membran der Karpule 2 durchsticht und somit eine Flüssigkeitsverbindung zur Kammer 9a herstellt. Geringfügiges weiteres Eindrehen des Karpulenhalters 7 führt zu einem weitem Vorschub der Stopfen 8a und 8b, so dass in der Kammer 9a befindliche Luft entweichen kann. Ein Vorschub wird normalerweise so lange durchgeführt, bis eine geringe Menge des Wirkstoffes 9a aus der Kanüle der Injektionsnadeleinheit austritt. Der Abschluss der Entlüftung kann durch ein taktiles, akustisches oder visuelles Signal angezeigt werden.

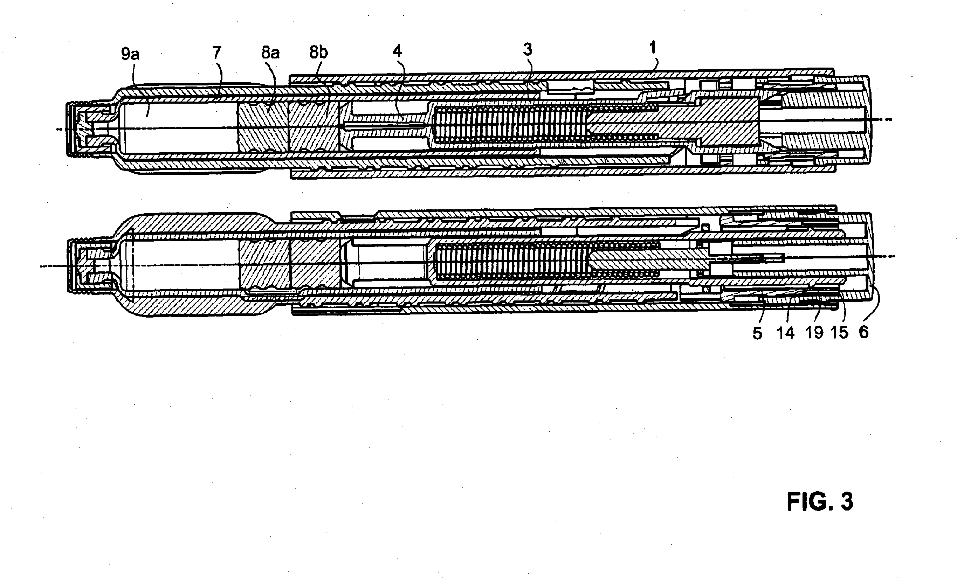

Dieser entlüftete Zustand ist in Figur 3 dargestellt. Auf die Darstellung der Injektionsnadeleinheit wurde aus Gründen der Einfachheit verzichtet. Bei der letzten Einschraubbewegung des Karpulenhalters 2 in das Gehäuse 1, bei dem auch die Karpule entlüftet werden kann, wird der Blockierring 5 von der Blockierposition in eine Freigabeposition bewegt. Wie in den Figuren 6a bis 6d gezeigt ist, weist hierfür der Karpulenhalter einen Anschlag 17 und der Blockierring einen Gegenanschlag 18 auf. Der Anschlag 17 des Karpulenhalters 2 ist derart ausgebildet, dass er bei der Drehbewegung des Karpulenhalters in Umfangsrichtung gegen den Gegenanschlag 18 des Blockierrings 5 anstösst. Bei einem weiteren Verdrehen des Karpulenhalters 2 nimmt der Karpulenhalter den Blockierring 5 mit, so dass dieser relativ zum Gehäuse 1 und zum Auslöseknopf 6 gedreht wird. Durch diese Drehbewegung wird der Blockierring von der Blockierposition in die Freigabeposition bewegt. Wie in Figur 3 dargestellt ist, wird bei der Drehung der Blockieranschlag 14 des Blockierrings 5 von dem Gegenanschlag 15 des Auslöseknopfes 6 weggedreht, bis dem Gegenanschlag 15 eine Nut oder Rille 19 des Blockierrings gegenüberliegt, innerhalb der der Gegenanschlag 15 des Auslöseknopfes 6 in Längsrichtung verschoben werden kann.

Beim Drehen des Blockierrings 5 mittels dem Karpulenhalter 2 werden zudem die Innenflächen des Blockierrings 5, welche die Schnapparme 12 an einem Ausschnappen aus ihrer Schnappposition hindern, aus dieser Position weggedreht. In der Freigabeposition des Blockierrings 5 liegen den Schnapparmen 12 Aussparungen in der Hülsenfläche des Blockierrings 5 gegenüber. Auch gegenüber den Schnapparmen 12 befindet sich der Blockierring 5 demnach in einer Freigabeposition.

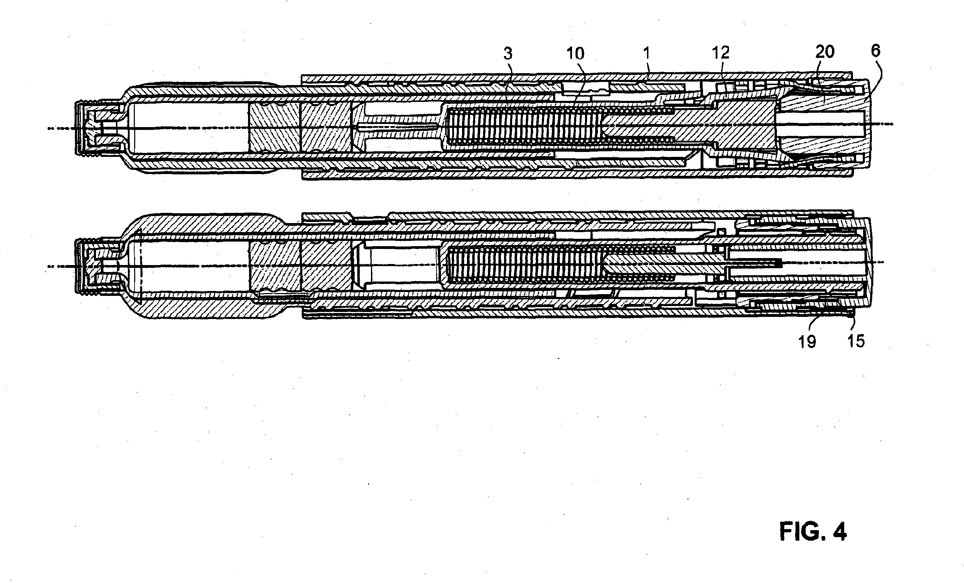

In Figur 4 ist die Verabreichungsvorrichtung in einem ausgelösten Zustand dargestellt, in dem der Auslöseknopf 6 relativ zum Gehäuse 1 entlang der Längsachse in das Gehäuse 1 eingedrückt wurde. Dabei wurden die Gegenanschläge 15 des Auslöseknopfes 6 innerhalb der Rillen 19 des Blockierrings 5 verschoben. Der Auslöseknopf 6 weist nach innen ragende Stege 20 auf, die in dem ausgelösten, bzw. eingeschobenen Zustand des Auslöseknopfes gegen schräge Flächen am proximalen Ende der Schnapparme 12 anliegen und diese beim Vorschub des Auslöseknopfes 6 radial nach aussen aufspreizen, so dass die Enden der Schnapparme innerhalb der Aussparungen im Blockierring 5 zu liegen kommen. Durch das Aufspreizen der Schnapparme 12 wird die Sicherung des Antriebsglieds 3 am Gehäuseelement 11 gelöst. In dem ausgelösten Zustand beginnt die Federkraft der Antriebsfeder 10 zu wirken und drückt gegen das Antriebsglied 3.

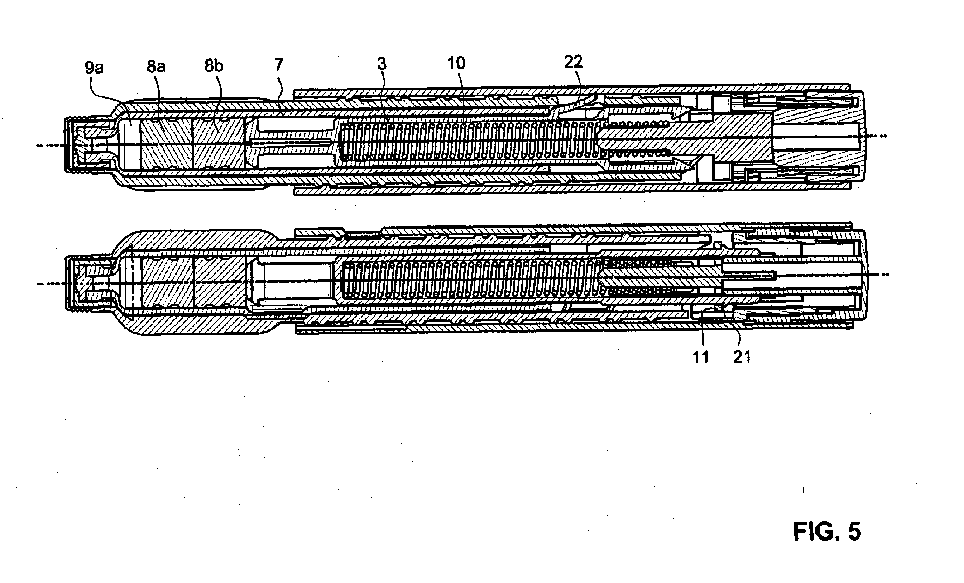

Wie in Figur 5 gezeigt ist, wird das Antriebsglied 3 durch die Kraft der Feder 10 relativ zur Karpule 7 vorgeschoben und treibt die Stopfen 8a und 8b innerhalb der Karpule 7 an, so dass der Wirkstoff aus der Kammer 8a ausgeschüttet wird. Die Antriebsfeder 10 schiebt das Antriebsglied 3 so lange in die Karpule vor, bis ein Vorsprung 21, der am Antriebsglied 3 vorgesehen ist, an einer Kante des Gehäuseelements 11 anschlägt. Sobald der Vorsprung 21 am Gehäuseelement 11 anschlägt, ist die Ausschüttung des Wirkstoffes beendet.

In dem dargestellten Ausfiihrungsbeispiel ist an dem Antriebsglied 3 ein flexibler Arm. 22 vorgesehen, der bei Beendigung der Ausschüttung in eine Aussparung im Karpulenhalter hineinragt und als Blockierung für eine Bewegung des Antriebsglieds 3 in proximaler Richtung dient. Ferner erzeugt der Arm 22 beim Einrasten in die Aussparung des Karpulenhalters ein akkustisches Geräusch, durch welches angezeigt wird, dass die Ausschüttung beendet ist.

In den Figuren 6a und 6b ist eine weitere Ausführungsform eines Verabreichungsgeräts mit einer erfindungsgemässen Halteeinrichtung gezeigt. In dieser Ausführungsform wird das Antriebsglied durch manuellen Druck angetrieben. Die Halteeinrichtung eines Antriebsglieds 3' weist seitlich an dem Antriebsglied 3' angeordnete Haltearme 4' auf, deren distales Ende gegenüber dem distalen Ende des Antriebsglieds 3' nach proximal zurückversetzt ist. Die Haltearme 4' schlagen ebenso wie im vorher beschriebenen Ausführungsbeispiels an die proximale Kante der Karpule 7, die im Karpulenhalter 2 eingebracht ist. Die Haltearme 4' weisen hierfür schräge Flächen auf, die an der Kante der Karpule anliegen, wie in Figur 6a zu sehen ist. Das Ende der Haltearme 4' weist nach innen in die Karpule 7 hinein. Sobald der Karpulenhalter 7 in das Gehäuse 1 eingeführt wird gleiten die schrägen Flächen der Haltearme 4' an der Kante der Karpule ab, so dass sich die Haltearme 4' radial in Richtung der Längsachse des Antriebsglieds verbiegen, wie in Figur 8b zu sehen ist. Die Haltearme 4' kommen in dieser Ausführungsform hinter einem Stössel 26 zu liegen, der am vorderen Ende des Antriebsglieds 3' angeordnet ist. Der Stössel 26 stösst gegen die Stopfen 8a und 8b, sobald der Karpulenhalter 2 in das Gehäuse 1 eingeführt ist. Durch weiteres Einführen, oder Eindrehen des Karpulenhalters in das Gehäuse werden die Stopfen mittels des Stössels 26 ebenso wie im vorher beschriebenen Ausführungsbeispiel innerhalb der Karpule 7 vorgeschoben.

Auch in dieser Ausführungsform wird die Karpule im Karpulenhalter zunächst durch den Anschlag der Haltearme 4' an der Kante der Karpule und anschliessend durch eine Klemmpassung zwischen den vorgespannten Haltearmen 4' und der Innenwand der Karpule 7 gehalten. Dadurch wird die Karpule 7 zu jedem Zeitpunkt bei der Anwendung der Verabreichungsvorrichtung in einer definierten Position gehalten.

### Bezugszeichenliste

- 1: Gehäuse
- 2: Karpulenhalter
- 3,3': Antriebsglied
- 4,4': Haltearm
- 5: Blockierring
- 6: Auslöseknopf
- 7: Zweikammer-Karpule
- 8a, b: Stopfen
- 9a, b: Kammer
- 10: Antriebsfeder
- 11: Gehäuseelement
- 12: Schnapparme
- 13: Schulter
- 14: Blockieranschlag
- 15: Gegenanschlag
- 16: Bypass
- 17, 17': Anschlag
- 18, 18': Gegenanschlag
- 19: Rille
- 20: Steg
- 21: Vorsprung
- 22: Arm
- 23: Verriegelungsarm
- 24: Blockieranschlag
- 25: Längsanschlag
- 26: Stössel

## Patentansprüche

1. Vorrichtung zur Verabreichung eines fluiden Produkts, die eine Aufnahmevorrichtung zur Aufnahme des fluiden Produkts (7) sowie ein Antriebsglied (3, 3') umfasst, das zur Verabreichung des fluiden Produkts in der Verabreichungsvorrichtung zwischen einer Ausgangsposition und einer Vorschubposition beweglich ist, wobei das Antriebsglieds (3, 3') in der Vorschubposition relativ zur Aufnahmevorrichtung (7) verschoben ist,
- wobei das Antriebsglied (3,3') eine Halteeinrichtung (4,4') aufweist, die die Aufnahmevorrichtung in einer definierten Position in der Verabreichungsvorrichtung hält,
- wobei die Halteeinrichtung (4,4') wenigstens einen axialen Anschlag für die Aufnahmevorrichtung (7) aufweist, um die Aufnahmevorrichtung (7) in der Ausgangsposition zu halten, wobei der axiale Anschlag eine schräge Fläche aufweist, die in distaler Richtung zur Längsachse geneigt ist, und
- wobei die Halteeinrichtung (4,4') in der Vorschubposition eine Klemmpassung mit der Aufnahmevorrichtung (7) eingeht, um die Aufnahmevorrichtung (7) in der Vorschubposition zu halten,
**dadurch gekennzeichnet, dass** die Halteeinrichtung zumindest einen von dem Antriebsglied (3,3') abragenden, flexibel beweglichen Haltearm (4,4') aufweist, der in der Ausgangsposition von einer Längsachse des Antriebsglied (3,3') seitlich abragt und der in der Ausgangsposition und in der Vorschubposition an der Aufnahmevorrichtung (7) angreift.

2. Vorrichtung zur Verabreichung eines fluiden Produkts nach Anspruch 1, **dadurch gekennzeichnet, dass** das Antriebsglied (3,3') und die Halteeinrichtung (4,4') einstückig ausgebildet sind.

3. Vorrichtung zur Verabreichung eines fluiden Produkts nach einem der vorhergehenden Ansprüche, **dadurch gekennzeichnet, dass** ein Gehäuse (1) und eine Aufnahmevorrichtung (2) zur Aufnahme eines Behälters (7) für das fluide Produkt vorgesehen sind, wobei die Aufnahmevorrichtung (2) mit dem Gehäuse (1) verbunden oder verbindbar ist und die Halteeinrichtung (4,4') den Behälter (7) in einer definierten Position in der Aufnahmevorrichtung (2) hält.

4. Vorrichtung zur Verabreichung eines fluiden Produkts nach Anspruch 3, **dadurch gekennzeichnet, dass** die Halteeinrichtung (4,4') in der Ausgangsposition des Antriebsglieds (3,3') an einem proximalen Ende des Behälters (7) angreift.

5. Vorrichtung zur Verabreichung eines fluiden Produkts nach einem der Ansprüche 3 oder 4, **dadurch gekennzeichnet, dass** die Halteeinrichtung (4, 4') in der Vorschubposition des Antriebsglieds (3,3') an einem Stopfen (8b) in dem Behälter (7) anschlägt.

6. Vorrichtung zur Verabreichung eines fluiden Produkts nach einem der vorhergehenden Ansprüche, **dadurch gekennzeichnet, dass** die Aufnahmevorrichtung (2) einen Gegenanschlag (13) aufweist, an dem der Behälter (7) mit seinem distalen Ende anschlägt.

## Claims

1. A device for administering a fluid product which includes a receiving device for receiving the fluid product (7) and a drive member (3, 3') which is moveable between a starting position and an advance position for administering the fluid product in the administration device, wherein the drive member (3, 3') is displaced in the advance position relative to the receiving device (7),
- wherein the drive member (3, 3') has a holding device (4, 4') which holds the receiving device in a defined position in the administration device,
- wherein the holding device (4, 4') has at least one axial abutment for the receiving device (7) to hold the receiving device (7) in the starting position, wherein the axial abutment has an inclined surface which is inclined in the distal direction relative to the longitudinal axis, and
- wherein the holding device (4, 4') in the advance position involves a clamping fit with the receiving device (7) to hold the receiving device (7) in the advance position,
**characterised in that** the holding device has at least one flexibly moveable holding arm (4, 4') which projects from the drive member (3, 3') and which in the starting position projects laterally from a longitudinal axis of the drive member (3, 3') and which engages the receiving device (7) in the starting position and in the advance position.

2. A device for administering a fluid product according to claim 1 **characterised in that** the drive member (3, 3') and the holding device (4, 4') are in one piece.

3. A device for administering a fluid product according to one of the preceding claims **characterised in that** there are provided a housing (1) and a receiving device (2) for receiving a container (7) for the fluid product, wherein the receiving device (2) is connected or connectable to the housing (1) and the holding device (4, 4') holds the container (7) in a defined position in the receiving device (2).

4. A device for administering a fluid product according to claim 3 **characterised in that** the holding device (4, 4') engages a proximal end of the container (7) in the starting position of the drive member (3, 3').

5. A device for administering a fluid product according to one of claims 3 and 4 **characterised in that** the holding device (4, 4') butts against a stopper (8b) in the container (7) in the advance position of the drive member (3, 3').

6. A device for administering a fluid product according to one of the preceding claims **characterised in that** the receiving device (2) has a counterpart abutment (13) against which the container (7) butts with its distal end.

## Revendications

1. Dispositif pour l'administration d'un produit fluide qui comprend un dispositif de réception pour la réception du produit fluide (7) ainsi qu'un organe d'entraînement (3, 3') qui est mobile pour l'administration du produit fluide dans le dispositif d'administration entre une position de départ et une position d'avance, l'organe d'entraînement (3, 3') étant décalé dans la position d'avance par rapport au dispositif de réception (7),
- dans lequel l'organe d'entraînement (3, 3') présente un dispositif de retenue (4, 4') qui maintient le dispositif de réception dans une position définie dans le dispositif d'administration,
- dans lequel le dispositif de retenue (4, 4') présente au moins une butée axiale pour le dispositif de réception (7) afin de maintenir le dispositif de réception (7) dans la position de départ, la butée axiale présentant une surface oblique qui est inclinée dans la direction distale par rapport à l'axe longitudinal, et
- dans lequel le dispositif de retenue (4, 4') entre dans la position d'avance dans un ajustement serré avec le dispositif de réception (7) afin de maintenir le dispositif de réception (7) dans la position d'avance,
**caractérisé en ce que** le dispositif de retenue présente au moins un bras de retenue (4, 4') mobile de manière flexible, faisant saillie de l'organe d'entraînement (3, 3'), qui, dans la position de départ, dépasse latéralement d'un axe longitudinal de l'organe d'entraînement (3, 3') et qui coopère dans la position de départ et dans la position d'avance avec le dispositif de réception (7).

2. Dispositif pour l'administration d'un produit fluide selon la revendication 1, **caractérisé en ce que** l'organe d'entraînement (3, 3') et le dispositif de retenue (4, 4') sont réalisés d'un seul tenant.

3. Dispositif pour l'administration d'un produit fluide selon l'une quelconque des revendications précédentes, **caractérisé en ce qu'**un boîtier (1) et un dispositif de réception (2) pour la réception d'un récipient (7) pour le produit liquide sont prévus, le dispositif de réception (2) étant ou pouvant être relié au boîtier (1) et le dispositif de retenue (4, 4') maintenant le récipient (7) dans une position définie dans le dispositif de réception (2).

4. Dispositif pour l'administration d'un produit fluide selon la revendication 3, **caractérisé en ce que** le dispositif de retenue (4, 4') coopère dans la position de départ de l'organe d'entraînement (3, 3') avec une extrémité proximale du récipient (7).

5. Dispositif pour l'administration d'un produit fluide selon l'une quelconque des revendications 3 ou 4, **caractérisé en ce que** le dispositif de retenue (4, 4') bute dans la position d'avance de l'organe d'entraînement (3, 3') contre un bouchon (8b) dans le récipient (7).

6. Dispositif pour l'administration d'un produit fluide selon l'une quelconque des revendications précédentes, **caractérisé en ce que** le dispositif de réception (2) présente une butée antagoniste (13), contre laquelle bute le récipient (7) avec son extrémité distale.
